(19) Europäisches Patentamt
European Patent Office
Office européen des brevets

(11) **EP 1 862 227 A2**

(12) **EUROPEAN PATENT APPLICATION**

(43) Date of publication:
**05.12.2007 Bulletin 2007/49**

(51) Int Cl.:
***B06B 1/06*** *(2006.01)*          ***G10K 11/02*** *(2006.01)*

(21) Application number: **07005154.5**

(22) Date of filing: **13.03.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **31.05.2006 JP 2006152765**
**27.02.2007 JP 2007047734**

(71) Applicant: **KABUSHIKI KAISHA TOSHIBA**
**Tokyo 105-8001 (JP)**

(72) Inventors:
• **Yamachita, Yohachi**
**Minato-ku**
**Tokyo 105-8001 (JP)**
• **Hosono, Yasuharu**
**Minato-ku**
**Tokyo 105-8001 (JP)**

(74) Representative: **HOFFMANN EITLE**
**Patent- und Rechtsanwälte**
**Arabellastrasse 4**
**81925 München (DE)**

(54) **Array ultrasonic probe and ultrasonic diagnostic apparatus**

(57)     Disclosed is an array ultrasonic probe (1), including a plurality of channels (3) arranged with space (4), each channel (3) having a piezoelectric element (6) and a laminated acoustic matching layer ($7_1$ to $7_3$) structure formed of at least three layers and arranged on the piezoelectric element (6), a backing (2) on which the piezoelectric element (6) of each channel (3) is mounted and having trenches (5) in which are formed at places corresponding to the spaces (4), and an acoustic lens (10) formed to cover at least the surface of the uppermost acoustic matching layer ($7_3$) of each channel (3), characterized in that the uppermost acoustic matching layer ($7_3$) comprises a silicone resin-containing mixture having a Shore hardness D not lower than 40 at 25°C, and exhibits an acoustic impedance of 1.8 to 2.5 MRayls at 25°C.

FIG.1

Printed by Jouve, 75001 PARIS (FR)

EP 1 862 227 A2

**Description**

The present invention relates to an array ultrasonic probe for transmitting ultrasonic signals to, for example, an object and for receiving the ultrasonic signals reflected from the object and to an ultrasonic diagnostic apparatus comprising the array ultrasonic probe.

A medical ultrasonic diagnostic apparatus or an ultrasonic image inspecting apparatus transmits ultrasonic signals to an object and forms an image showing the inner region of the object by receiving the reflected signals (echo signals) coming from within the object. An electronically operated array ultrasonic probe capable of transmitting-receiving ultrasonic signals is used mainly in the medical ultrasonic diagnostic apparatus or the ultrasonic image inspecting apparatus.

The array ultrasonic probe comprises a backing, a plurality of channels mounted to the backing and arranged a prescribed distance apart from each other to form an array, and an acoustic lens mounted to the channels. Each of the plural channels comprises a piezoelectric element prepared by mounting electrodes to both surfaces of a piezoelectric body consisting of, for example, a lead zirconium titanate (PZT) based piezoelectric ceramic material or a relaxor based single crystal material and an acoustic matching layer formed on the piezoelectric element. Incidentally, it is possible for a trench to be formed in some cases in the backing in a manner to correspond to the space between adjacent channels.

For operating the array ultrasonic probe of the construction described above so as to carry out the required diagnosis, the piezoelectric element for each channel is driven under the state that the acoustic lens of the ultrasonic probe is allowed to abut against the object so as to permit the ultrasonic signals to be transmitted from the front surface of the piezoelectric element into the object, e.g., the human body. The ultrasonic signals are converged onto a desired position within the object by the electronic focus depending on the drive timing of the piezoelectric element and the focus by the acoustic lens. In this case, it is possible to transmit ultrasonic signals into a desired range within the object by controlling the drive timing of the piezoelectric element so as to make it possible to obtain an ultrasonic image (tomographic image) having a desired range by receiving and processing the echo signals reflected from the object. In the driving of the piezoelectric element included in the ultrasonic probe, ultrasonic signals are also released to the back side of the piezoelectric element. Therefore, a backing is arranged on the back side of the piezoelectric element for each channel so as to allow the backing to absorb and damp the ultrasonic signals transmitted to the back side, thereby avoiding the ultrasonic reflection that the normal ultrasonic signals are transmitted into the object together with the ultrasonic signals (reflected signals) coming from the back side.

An acoustic matching layer of a single layer structure, a two-layer structure or a multi-layered inclined structure having at least three layers is known to the art. It is described in " T. Inoue et al., IEEE, UFFC, vol. 34 No. 1, 1987, pp. 8-15" that an acoustic matching layer having at least three layers is suitably used for obtaining a large bandwidth.

On the other hand, a general method of manufacturing an ultrasonic probe is disclosed in JP-A 2005-198261 (KOKAI). To be more specific, it is described that a piezoelectric element prepared by forming electrodes on both surfaces of a piezoelectric body consisting of a piezoelectric material such as PZT is attached to a rubber plate used as a backing, followed by further bonding an acoustic matching layer to the piezoelectric element so as to obtain a laminate structure. In this bonding process, a heat treatment is applied in some cases at 80 to 150°C so as to cure the organic adhesive layer. Such being the situation, it is necessary for the acoustic matching layer to exhibit a resistance to heat. In the next step, the laminate structure is cut by a dicer at a width of about 50 to 300 $\mu$m (array cutting) such that the array cutting starts from the side of the acoustic matching layer. By the array cutting of the acoustic matching layer, the crosstalk between adjacent channels is prevented. Therefore, it is important for the array cutting of the acoustic matching layer to be performed with a high dicing machinability. Then, the dicing trench between the adjacent channels is loaded with a relatively soft resin such as a silicone rubber having a low acoustic impedance and high damping properties so as to permit the channels to maintain a high mechanical strength. Further, an acoustic lens is bonded to the upper surfaces of the acoustic matching layers for a plurality of channels so as to obtain a desired ultrasonic probe.

The acoustic lens is formed of a mixed material obtained by adding an inorganic filler to a silicone rubber. To be more specific, a mixed material exhibiting an acoustic impedance of 1.3 to 1.7 MRayls at room temperature of 25°C is used for preparing the acoustic lens. The acoustic lens exhibits a thermal expansion coefficient of about 200 ppm/°C under the temperature ranging between room temperature and 40°C. A silicone rubber-based adhesive or a modified silicone rubber-based adhesive is used for bonding the acoustic lens to the acoustic matching layer.

In the driving stage of the array ultrasonic probe of the construction described above, the ultrasonic energy emitted from the piezoelectric elements of a plurality of channels is absorbed and damped by the acoustic matching layers and the acoustic lens. Since the ultrasonic energy is partly converted into heat in this stage, it is possible for the temperature of the acoustic matching layers to be elevated to exceed 60°C in, for example, an ultrasonic probe for a circulatory organ. It should also be noted that a considerably high pressure is kept applied to the acoustic matching layers during operation of the ultrasonic probe. Because of the difference in the thermal expansion and the mechanical pressure between the acoustic lens and the acoustic matching layer, which is produced by the thermal effects pointed out above, it is possible for the acoustic lens to peel off from the uppermost acoustic matching layer and for the uppermost acoustic matching layer to peel off from the acoustic matching layer in contact with the uppermost acoustic matching layer. As a result, a nonuniformity of sensitivity is generated within the ultrasonic probe, which lowers the reliability of the ultrasonic probe. In an extreme case, the ultrasonic probe ceases to function. Particularly, the phenomenon pointed out above is serious in the case where the uppermost acoustic matching layer is formed of an epoxy resin and the acoustic lens is formed of a silicone resin, because the difference in thermal expansion coefficient between the epoxy resin and the silicone rubber is increased to reach a high level of 150 ppm/°C.

The properties of the acoustic matching layer used in the ultrasonic probe are specifically exemplified in JP-A 2005-198261 (KOKAI) referred to previously. For example, the uppermost acoustic matching layer in contact with the acoustic lens is taken up in this prior art from among a plurality of acoustic matching layers. It is described that a material having an acoustic impedance closer to the acoustic impedance (1.4 to 1.6 MRayls) of the human body is used for preparing the uppermost acoustic matching layer in contact with the acoustic lens.

It was customary in the past to use a material based on a polyurethane rubber, polyethylene, a silicone rubber or an epoxy resin for manufacturing the acoustic matching layer described above. To be more specific, JP-A 10-75953 (KOKAI) teaches that, in order to decrease the amount of heat transmitted from the heat generating body inside the ultrasonic probe to the surface of, for example, the living body, an acoustic matching layer having a sufficiently low thermal conductivity is used as at least one of the acoustic matching layers arranged between the piezoelectric element and the surface of the living body. It is described that the acoustic matching layer having a sufficiently low thermal conductivity is prepared by dispersing fine particles having a low thermal conductivity such as fine particles of a silicone resin in a base material consisting of, for example, an epoxy resin. Also, an acoustic matching layer of the construction that a polyurethane resin is loaded with a polyethylene fiber or a carbon fiber is disclosed in Toshio Kondo and Hiroyuki Fujimoto, Proceedings 2003 IEEE Ultrasonic Symposium p. 1318-1321 and Toshio Kondo, Mitsuyoshi Kitatuji and Mikio Izumi, Proceedings, 2004 IEEE Ultrasonic Symposium p. 1659-1662". However, these materials do not satisfy all of the various properties required for the acoustic matching layer including, for example, the low acoustic attenuation factor, the good dicing machinability, the high heat resistance, the good adhesivity to the upper or lower layer, and the good coincidence of the thermal expansion coefficient with the acoustic lens. In addition, the materials exemplified above do not satisfy all the appropriate acoustic impedances.

U.S.P. 5,505,205 discloses a material of an acoustic lens for an ultrasonic probe having a high hardness. It is indicated in Table II of the US Patent document quoted above that trimethylsiloxymethacryloxy propylsilane (TRIS) exhibits an acoustic velocity of about 1650 m/s. However, it is not described nor even remotely suggested in this US Patent document that the acoustic lens material exemplified above can be applied to an acoustic matching layer. Also, even if methyl methacrylate (Example 1) or tetrabutyl styrene (Example II) described in US patent document is applied, it is impossible to obtain acoustic properties required for the acoustic matching layer such as the acoustic velocity, the acoustic attenuation factor and the acoustic impedance. In addition, the materials exemplified above are not excellent in, for example, the dicing machinability as the acoustic matching layer, the heat resistance, the adhesivity to the upper or lower layer and the coincidence of the thermal expansion coefficient with the acoustic lens.

Also, each of JP-A 2004-339482 (KOKAI) and JP-A 2005-272697 (KOKAI) discloses a material used for manufacturing a Laser Emission Diode (LED). It is taught that the material contains a silicone resin having a high hardness. However, the information on the acoustic properties of the silicone material for an LED, such as the acoustic velocity, the acoustic attenuation factor and the acoustic impedance is not disclosed in any of the patent documents quoted above. Also, the application of the silicone material to an ultrasonic probe is not referred to in any of the patent documents quoted above.

Therefore, an object of the present invention is to provide an array ultrasonic probe including an uppermost acoustic matching layer which exhibits a low acoustic attenuation factor, a high adhesivity to the silicone series adhesive layer or the epoxy series adhesive layer interposed between the upper and lower layers, and is excellent in the dicing workability and excellent in heat resistance.

Another object of the present invention is to provide an ultrasonic diagnostic apparatus including the array ultrasonic probe.

Namely, according to first aspect of the present invention, there is provided an array ultrasonic probe, comprising:

a plurality of channels arranged with spaces, each channel including a piezoelectric element and acoustic matching layers formed on the piezoelectric element and having at least three layers;
a backing on which the piezoelectric element of each channel is mounted and having trenches in which are formed at places corresponding to the spaces; and
an acoustic lens formed to cover at least the surface of the uppermost acoustic matching layer of each channel;

characterized in that the uppermost acoustic layer comprises a silicone resin-containing mixture having a Shore hardness D not lower than 40 at 25°C, and exhibits an acoustic impedance of 1.8 to 2.5 MRayls.

According to second aspect of the present invention, there is provided an ultrasonic diagnostic apparatus, comprising:

an array ultrasonic probe including a plurality of channels arranged with spaces, each channel having a piezoelectric element and a laminated acoustic matching layer structure formed of at least three layers and arranged on the piezoelectric element; a backing on which the piezoelectric element of each channel is mounted and having trenches in which are formed at places corresponding to the spaces; and an acoustic lens formed to cover at least the surface of the uppermost acoustic matching layer of each channel, characterized in that the uppermost acoustic matching layer comprises a silicone resin-containing mixture having a Shore hardness D not lower than 40 at 25°C, and exhibits an acoustic impedance of 1.8 to 2.5 MRayls at 25°C; and
an ultrasonic probe controller connected to the ultrasonic probe via a cable.

The invention can be more fully understood from the following detailed description when taken in conjunction with the accompanying drawings, in which:

FIG. 1 is an oblique view showing the construction in the gist portion of the array ultrasonic probe according to an embodiment;
FIG. 2 is a cross-sectional view showing the construction in the gist portion of the ultrasonic probe shown in FIG. 1;
FIG. 3 is a cross-sectional view schematically showing the construction of a third acoustic matching layer incorporated in the array ultrasonic probe according to an embodiment;
FIG. 4 is a cross-sectional view schematically showing another construction of a third acoustic matching layer incorporated in the array ultrasonic probe according to an embodiment;
FIG. 5 schematically shows the construction of an ultrasonic diagnostic apparatus according to an embodiment;
FIG. 6 is a graph showing the Thermogravimetric/Differential Thermal Analysis (TG/DTA) curve of a cured material of the silicone resin-containing mixture (raw material of the third acoustic matching layer) obtained in Example 1;
FIG. 7 shows the Fourier Transform Infrared Spectroscopy (FTIR) spectrum of the cured material of the silicone resin-containing mixture obtained in Example 1 (raw material of the third acoustic matching layer);
FIG. 8 is a graph showing the TG/DTA curve the cured material of a general-purpose silicone resin;
FIG. 9 shows the FTIR spectrum of the cured material of the general-purpose silicone resin;
FIG. 10 is a graph showing the relationship between the acoustic impedance and the Shore hardness D in the material of the third acoustic matching layer in respect of Example 1 and each of Comparative Examples 1 to 5; and
FIG. 11 is a graph showing the relationship between the acoustic impedance and the acoustic attenuation factor in the material of the third acoustic matching layer in respect of Example 1 and each of Comparative Examples 1 to 5.

An array ultrasonic probe and an ultrasonic diagnostic apparatus according to an embodiment of the present invention will now be described with reference to the accompanying drawings.

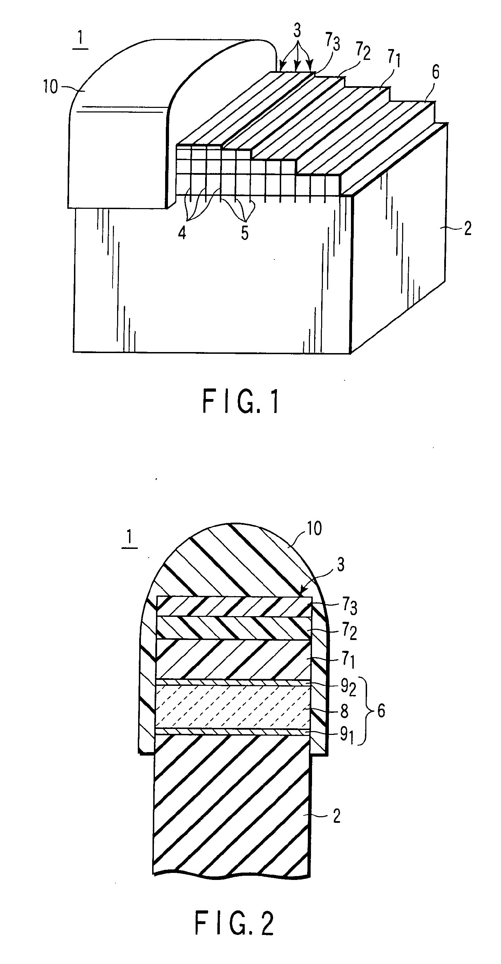

FIG. 1 is an oblique view showing the construction in the gist portion of an array ultrasonic probe according to an embodiment, and FIG. 2 is a cross sectional view partly showing the construction of the array ultrasonic probe shown in FIG. 1.
As shown in the drawings, the array ultrasonic probe 1 comprises a backing 2. A plurality of channels 3 are arranged on the backing 2 with space 4. Trenches 5 are formed in the backing 2 in a manner to correspond to the spaces 4 between the adjacent channels 3. Incidentally, it is possible for the spaces 4 to be loaded with a relatively soft resin such as silicone rubber having high damping properties so as to permit the channels 3 to maintain a desired mechanical strength. It is possible for the resin noted above to be loaded not only in the spaces 4 but also in the trenches 5 formed in the backing 2 and positioned below the spaces 4.
Each channel 3 comprises a piezoelectric element 6 and a plurality of acoustic matching layers formed on the piezoelectric element 6. For example, three (first to third) acoustic matching layers $7_1$ to $7_3$ are formed on the piezoelectric element 6. As shown in FIG. 2, the piezoelectric element 6 comprises a piezoelectric body 8 consisting of, for example, a lead zirconium titanate (PZT) based piezoelectric ceramic material or a relaxor based single crystal material as well as first and second electrodes $9_1$ and $9_2$ formed on the both surfaces of the piezoelectric body 8. The first electrode $9_1$ of the piezoelectric element 6 is bonded and fixed to the backing 2 by using, for example, an epoxy resin based adhesive layer (not shown). The first acoustic matching layer $7_1$ is bonded and fixed to the second electrode $9_2$ of the piezoelectric element 6 by using, for example, an epoxy resin based adhesive layer (not shown). The second acoustic matching layer $7_2$ is bonded and fixed to the first acoustic matching layer $7_1$ by using, for example, an epoxy resin based adhesive layer (not shown). Further, the third acoustic matching layer $7_3$ is bonded and fixed to the second acoustic matching layer $7_2$ by using, for example, an epoxy resin based adhesive layer (not shown).
An acoustic lens 10 is formed to extend downward from the upper surface of the uppermost third acoustic matching layer $7_3$ of the channel 3 so as to cover the side surfaces of the third, second and first acoustic matching layers $7_3$, $7_2$ and $7_1$ and to further cover the side surface of the piezoelectric element 6 and the side surface of the backing 2 that is positioned in the vicinity of the piezoelectric element 6. The acoustic lens 10 is bonded and fixed to the surface of the third acoustic matching layer $7_3$ by using a rubber-based adhesive layer (not shown). It is desirable for the rubber-based adhesive to be formed of a modified silicone-based adhesive having an acoustic impedance of 1.3 to 1.8 MRayls at 25°C.

The backing 2, the plural channels 3 and the acoustic lens 10 are housed in a case (not shown). Also housed in the case are a control circuit for controlling the drive timing of the piezoelectric element 6 of each channel 3 and a signal processing circuit (not shown) including an amplifying circuit for amplifying the signal received by the piezoelectric element 6. An extending signal line and ground line (not shown) are connected to the first and second electrodes $9_1$, $9_2$ of the piezoelectric element 6 and extend from the case on the opposite side of the acoustic lens 10 to the outside so as to be connected to the control circuit (not shown). Incidentally, it is possible for the signal line to be connected to the second electrode $9_2$ of the piezoelectric element 6 and for the ground line to be connected to the junction between the second acoustic matching layer $7_2$ and the third acoustic matching layer $7_3$.

In the array ultrasonic probe of the construction described above, voltage is applied between the first and second electrodes $9_1$ and $9_2$ of the piezoelectric element 6 in each channel 3 so as to cause the piezoelectric body 8 to resonate, with the result that an ultrasonic wave is radiated (transmitted) through the acoustic matching layers (first to third acoustic matching layers $7_1$, $7_2$ and $7_3$) of each channel 3 and the acoustic lens 10. When the ultrasonic wave is received, the piezoelectric body 8 of the piezoelectric element 6 in each channel 3 is vibrated by the ultrasonic wave received through the acoustic lens 10 and the acoustic matching layers of each channel (first to third acoustic matching layers $7_1$, $7_2$ and $7_3$). Then, the vibration is converted into an electric signal so as to obtain an image.

The third acoustic matching layer (uppermost acoustic matching layer) $7_3$ comprises a silicone resin-containing mixture having a Shore hardness D not smaller than 40 at 25°C, and exhibits an acoustic impedance of 1.8 to 2.5 MRayls at 25°C. The Shore hardness D is obtained in accordance with, for example, JIS K 6253 by using Durometer type D. It is desirable for the third acoustic matching layer (uppermost acoustic matching layer) $7_3$ to exhibit an acoustic attenuation factor not larger than 5 dB/cm/MHz as measured with 5 MHz at 25°C and to exhibit a acoustic attenuation factor index, which is a product of the acoustic attenuation factor and the acoustic velocity and which is not larger than 900 m/s · dB/mm/MHz.

The silicone resin-containing mixture noted above denotes a two-part mixture consisting of, for example, an organo-polysiloxane and a silicone-based mixture containing a silicone modified organic resin of epoxy-terminated.

The organopolysiloxane noted above includes, for example, a material of organopolysiloxane containing a methyl group or a vinyl group. Also, the silicone modified organic resin compound noted above denotes, for example, a block copolymer between a silicone resin and an organic resin. The particular block copolymer can be obtained by the reaction between a modified silicone intermediate and an organic resin having a functional group. Various silicone modified resins can be obtained depending on the kinds of the organic resins used. Typical silicone modified resins include an alkyd silicone and an epoxy silicone.

It is a preferable that the silicone resin-containing mixture has the C-H stretching vibration of benzene nucleus and the C-H outside planar angle change vibration in a spectrum obtained by a measuring of the Fourier Transform Infrared Spectrophotometer.

It is possible to use, for example, SCR 1004, SCR 1011, and SCR 1012, which are silicone resin-containing mixtures for LEDs, available from Shin-Etsu Chemical Co. Ltd., as the silicone resin-containing mixtures noted above. It is also possible to use DE 6665, available from Dowcorning Inc., as the silicone resin-containing mixtures exhibiting the properties noted above. Among the silicone resin-containing mixtures exemplified above, it is particularly desirable to use SCR 1011, which has a low acoustic attenuation factor.

Also, it is possible to use as the silicone resin-containing mixture noted above a two-part mixture consisting of, for example, an organopolysiloxane and a silicone-based mixture containing an alkyl silicone compound. The alkyl silicone compound noted above includes, for example, SR 2107 available from Toray-Dowcorning Inc.

It is possible for the third acoustic matching layer (uppermost acoustic matching layer) $7_3$ to be formed of the silicone resin-containing mixture alone having the properties noted above. Further, in order to improve the adhesivity, the dicing machinability and the heat resistance, it is possible to add a filler to the silicone resin-containing mixture used for manufacturing the third acoustic matching layer $7_3$.

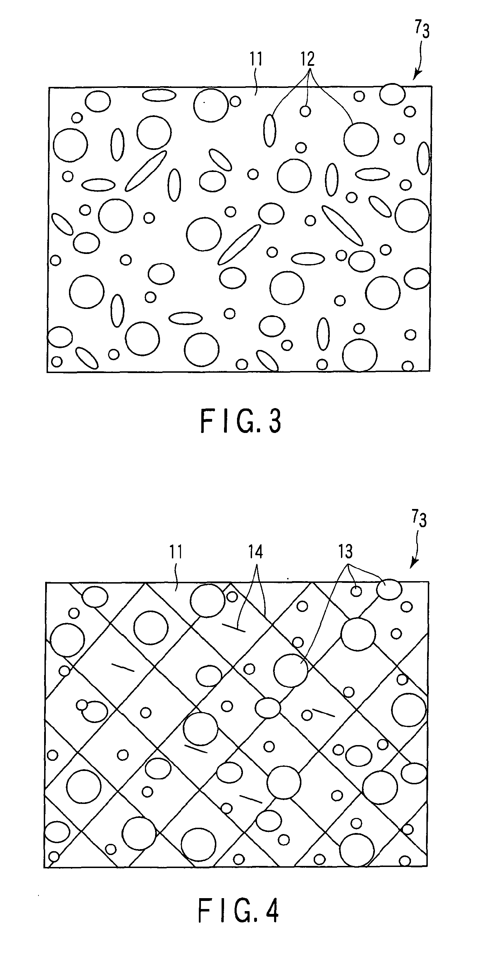

The uppermost acoustic matching layer $7_3$ formed of a composite material prepared by adding a filler to a silicone resin-containing mixture is constructed as shown in, for example, FIG. 3 or FIG. 4.

The acoustic matching layer $7_3$ shown in FIG. 3 comprises a matrix 11 of a silicone resin-containing mixture and organic filler particles 12 loaded in the matrix 11 in an amount not larger than 30% by volume. The uppermost acoustic matching layer, in which organic filler particles are dispersed in a silicone resin-containing mixture, exhibits an acoustic velocity (longitudinal wave) of 1750 to 2200 m/s at 25°C.

At least one kind of particles selected from the group consisting of, for example, silicone rubber particles, fluorocarbon resin particles and urethane rubber particles can be used as the organic filler particles. The organic filler particles can be used singly or in the form of a mixture of different kinds of particles. The organic filler particles produce the effect of controlling the acoustic velocity. The organic filler particles are shaped, for example, spherical. It is desirable for the spherical organic filler particles to have an average diameter of, for example, 1 to 10 $\mu$m.

If the amount of the organic filler particles contained in the matrix 11 of the silicone resin-containing mixture exceeds 30% by volume, the acoustic attenuation factor of the acoustic matching layer $7_3$ tends to be increased. Also, the mechanical strength of the acoustic matching layer is lowered, with the result that the dicing machinability of the acoustic matching layer tends to be lowered. It is more desirable for the organic filler particles to be dispersed in the silicon resin matrix in an amount not larger than 10% by volume.

The acoustic matching layer $7_3$ shown in FIG. 4 comprises a matrix 11 of a silicone resin-containing mixture and an inorganic filler dispersed in the silicon resin matrix 11 in an amount not larger than 10% by volume. The inorganic filler noted above has a density not higher than 6 g/cm$^3$ and includes, for example, spherical silicon oxide particles 13 and inorganic fibers 14. It is possible to control easily the particular acoustic matching layer to exhibit a acoustic attenuation factor not larger than 5 dB/cm/MHz as measured with 5 MHz at 25°C and to exhibit the acoustic attenuation factor index (i.e., the product of the acoustic attenuation factor and the acoustic velocity), which is not larger than 900 m/s·dB/mm/MHz. It should also be noted that the processing strength, the adhesivity and the heat resistance of the acoustic matching layer having an inorganic filler added thereto can be effectively improved, compared with those of the acoustic matching layer not having an inorganic filler added thereto.

The inorganic filler is, for example, powdery or fibrous. It is possible for the powdery or fibrous inorganic filler to be contained in the silicone resin-containing mixture singly or in the form of a mixture.

The powdery inorganic filler includes, for example, a zinc oxide powder, a zirconium oxide powder, an alumina powder, a silica powder such as an aerosil silica, a titanium oxide powder, a silicon carbide powder, an aluminum nitride powder, a carbon powder and a boron nitride powder. The powdery inorganic filler can be used singly or in the form of a mixture. It is desirable for the powdery inorganic filler to have an average particle diameter not larger than 0.5 $\mu$m, more preferably not larger than 0.1 $\mu$m. The uppermost acoustic matching layer $7_3$, in which such a fine powdery inorganic filler is further dispersed in a silicone resin-containing mixture, makes it possible to obtain an excellent dicing machinability while maintaining the acoustic attenuation factor at a low level.

The fibrous inorganic filler includes, for example, a carbon fiber, a silicon carbide fiber, a zinc oxide fiber, an alumina fiber and a glass fiber. It is possible to use the fibrous inorganic filler singly or in the form of a mixture.

Particularly, it is desirable to use a glass fiber as the fibrous inorganic filler. The glass fiber includes, for example, a quartz glass fiber and a soda glass fiber. It is also possible to use an electrically conductive carbon fiber. The carbon fiber includes carbon fibers of various grades such as a pitch-series carbon fiber and a PAN-series carbon fiber. Incidentally, the fibrous inorganic filler is not limited to the filler formed of a single kind of material. It is also possible to use a fibrous inorganic filler prepared by coating the surface of, for example, a carbon fiber with a resin.

It is desirable for the fibrous inorganic filler to have a diameter not larger than 10 $\mu$m and a length that is at least 5 times as much as the diameter. The acoustic matching layer containing a small amount of the fibrous inorganic filler of the size noted above makes it possible to set the acoustic attenuation factor, which is measured with 5 MHz, at a level not higher than 5 dB/cm/MHz. It follows that it is possible to transmit-receive the ultrasonic signal while preventing the ultrasonic signal from being deteriorated by the acoustic matching layer. Also, a sufficient strength required in the stage of the dicing process is imparted to the acoustic matching layer. In addition, it is possible to improve further the heat resistance and the dicing machinability of the acoustic matching layer in the stage of the dicing process. In particular, it is possible to obtain an acoustic matching layer having a further lowered acoustic attenuation factor by using a fibrous inorganic filler having a diameter not larger than 5 $\mu$m. Also, it is possible to obtain an acoustic matching layer that is further improved in the heat resistance and the dicing machinability by using a fibrous inorganic filler having a length at least 20 times as much as the diameter.

If the amount of the inorganic filler dispersed in the matrix of the silicone resin-containing mixture exceeds 10% by volume, the acoustic attenuation factor of the ultrasonic wave is rapidly increased, with the result that the value of the acoustic impedance is increased to exceed 2.5 MRayls so as to make it impossible to obtain an acoustic matching having a low acoustic impedance. It is more desirable for the inorganic filler to be dispersed in the matrix of the silicone resin-containing mixture in an amount of 2 to 5% by volume.

Incidentally, it is possible to add the inorganic filler to the silicone resin-containing mixture together with the organic filler particles described previously.

Where the acoustic matching layer has a laminated three-layer structure, it is desirable for the acoustic matching layers other than the uppermost acoustic matching layer $7_3$ to have appropriate acoustic impedances. To be more specific, it is desirable for the lowermost acoustic matching layer in contact with the piezoelectric element 6 (i.e., first acoustic matching layer $7_1$) to have an acoustic impedance of 10 to 15 MRayls at 25°C, and for the intermediate acoustic matching layer (i.e., second acoustic matching layer $7_2$) to have an acoustic impedance of 2.7 to 8 MRayls at 25°C. In the acoustic matching layer having a laminated three-layer structure noted above, the acoustic velocity is changed depending on the thickness of each of the laminated acoustic matching layers. In the standard case, the uppermost acoustic matching layer $7_3$ has a thickness $\lambda/4$, where $\lambda$ denotes the wavelength of the ultrasonic wave. It is desirable for the thickness of λ/4 to be 30 to 200 μm.

How to manufacture the acoustic matching layer will now be described.

In the first step, two-part silicone resins, which are in the form of a liquid, are weighed at a prescribed ratio, followed by sufficiently mixing the weighed silicone resins. In the next step, the mixture is put in a polyethylene cup and is defoamed, followed by curing the mixture at room temperature to 85°C for 2 hours and, then, at 125°C for 2 hours, so as to manufacture an acoustic matching layer.

Incidentally, in manufacturing the acoustic matching layer, it is possible for the silicone resin to be mixed with at least one filler selected from the group consisting of the organic filler particles described previously and the inorganic filler described previously. In the case of using a fibrous inorganic filler as the filler, it is desirable for the fibrous inorganic filler to be subjected to, for example, a vacuum impregnation before the above-noted stage of curing the mixture. Where the mixture has a high viscosity, it is possible to lower the viscosity of the mixture by using an organic solvent such as normal hexane or toluene.

How to manufacture an ultrasonic probe according to an embodiment will now be described.

In the first step, a piezoelectric element, a first acoustic matching layer, a second acoustic matching layer and a third acoustic matching layer are laminated one upon the other in the order mentioned on a backing with an epoxy resin-based adhesive layer having a low viscosity interposed between the adjacent backings so as to obtain a laminate structure. In the next step, the laminate structure is baked at, for example, 120°C for about one hour so as to cure each of the epoxy resin-based adhesive layers, thereby bonding and fixing the backing to the piezoelectric element, bonding and fixing the piezoelectric element to the first acoustic matching layer, bonding and fixing the first acoustic matching layer to the second acoustic matching layer, and bonding and fixing the second acoustic matching layer to the third acoustic matching layer.

In the next step, the laminate structure is cut by, for example, a dicing blade from the third acoustic matching layer toward the backing. The laminate structure is cut at a width (pitch) of, for example, 50 to 200 μm so as to divide the laminate structure into an array of a plurality of channels each including the piezoelectric element, as well as the first, second and third acoustic matching layers. In this stage, trenches corresponding to the spaces between the adjacent channels are formed in the surface layer of the backing. In the next step, the spaces between the adjacent channels are loaded, if necessary, with a relatively soft resin such as a silicone resin having a low acoustic impedance and a high acoustic attenuation factor. Then, an acoustic lens is bonded and fixed to the third acoustic matching layer of each channel, followed by housing the backing, the plural channels and the acoustic lens in a case so as to manufacture a desired ultrasonic probe.

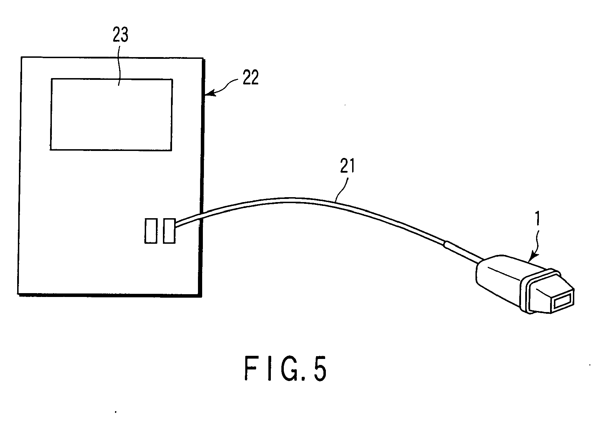

An ultrasonic diagnostic apparatus equipped with an ultrasonic probe according to an embodiment will now be described with reference to FIG. 5.

A medical ultrasonic diagnostic apparatus (or ultrasonic image inspecting apparatus), which transmits an ultrasonic signal to an object and receives a reflected signal (echo signal) from the object for forming an image of the object, comprises an array ultrasonic probe performing the function of transmitting-receiving an ultrasonic signal. The ultrasonic probe is constructed as shown in FIGS. 1 and 2. As shown in FIG. 5, the ultrasonic probe 1 is connected to an ultrasonic diagnostic apparatus body 22 via a cable 21. Arranged within the ultrasonic diagnostic apparatus body 22 are an ultrasonic probe controller (not shown) for enabling the ultrasonic probe to transmit an ultrasonic signal to an object and to receive the ultrasonic signal reflected from the object. Further, a display 23 is also arranged within the ultrasonic diagnostic apparatus body 22.

The array ultrasonic probes according to an embodiment described above are arranged apart from each other. Each of the array ultrasonic probes comprises a piezoelectric element, a plurality of channels each having a multi-layered acoustic matching layer formed on the piezoelectric element, a backing having the piezoelectric element of each channel mounted thereto and provided with a trench in a manner to correspond to the space between the adjacent channels, and an acoustic lens formed in a manner to cover at least the surface of the uppermost acoustic matching layer of each channel. It should be noted that the uppermost acoustic matching layer comprises a silicone resin-containing mixture having a Shore hardness D not smaller than 40 at 25°C and exhibits an acoustic impedance of 1.8 to 2.5 MRayls at 25°C. The array ultrasonic probe of the construction described above produces prominent effects as pointed out in the following:

(1) Since the uppermost acoustic matching layer exhibits a low acoustic attenuation factor and an appropriate acoustic impedance, it is possible to provide an array ultrasonic probe of a high performance, which permits effectively transmitting and receiving an energy of the ultrasonic wave.

(2) Since the uppermost acoustic matching layer is excellent in the dicing machinability, it is possible to form precisely a channel having a desired width by the dicing process performed by using, for example, a diamond saw. As a result, a high resolution ultrasonic probe is available due to a suppression of the crosstalk between each channels.

(3) The uppermost acoustic matching layer is excellent in heat resistance and exhibits a high adhesivity to the silicone based adhesive layer or the epoxy based adhesive layer interposed between the upper and lower layers (which are positioned between the acoustic lens and the lower acoustic matching layer). It follows that, even if the acoustic matching layer is heated by the absorption and damping of the ultrasonic wave or by the application of a mechanical pressure to the acoustic matching layer, it is possible to prevent the acoustic lens from being peeled off from the uppermost acoustic matching layer and to prevent the uppermost acoustic matching layer from being peeled off from the acoustic matching layer positioned immediately below the uppermost acoustic matching layer. As a result, it is possible to provide an array ultrasonic probe having a high reliability over a long period in respect of the uniformity in sensitivity among the channels.

Particularly, where the uppermost acoustic matching layer comprises a silicone resin matrix and a powdery inorganic filler having a density not higher than 6 g/cm$^3$, such as a silica powder, which is dispersed in the resin matrix in an amount not larger than 10% by volume, it is possible to further improve the uppermost acoustic matching layer in respect of the adhesivity to the epoxy based adhesive and the dicing machinability in the stage of the dicing process.

Also, by using a fibrous inorganic filler such as a glass fiber as the inorganic filler, it is possible to suppress the increase in the acoustic attenuation factor of the uppermost acoustic matching layer and to further improve the dicing machinability and the mechanical strength of the uppermost acoustic matching layer in the stage of the dicing process of the acoustic matching layer.

Further, where the uppermost acoustic matching layer is controlled to exhibit a thermal expansion coefficient such that the difference in thermal expansion coefficient between the acoustic lens and the uppermost acoustic matching layer is not larger than 50 ppm/°C, the uppermost acoustic matching layer is unlikely to peel off even if heat is repeatedly applied to the ultrasonic probe during operation of the ultrasonic probe. It follows that it is possible to obtain an ultrasonic probe having a high reliability.

The ultrasonic diagnostic apparatus according to an embodiment of the present invention comprises an array ultrasonic probe of high reliability, which is low level of crosstalk and high in performance. It follows that the ultrasonic diagnostic apparatus of the present invention permits improving the quality of the tomographic image and also permits improving the sensitivity.

The present invention will now be described more in detail with reference to Examples of the present invention.

(Example 1)

Two-part silicone resins of SCR 1011 resin A and SCR 1011 resin B, available from Shin-Etsu Chemical Co. Ltd., were weighed accurately at a weight ratio of 1:1. Then, the silicone resin-containing mixture was put in a polyethylene cup and uniformly mixed for 3 minutes while stirring the mixture within a rotary mixer. The liquid resin mixture was defoamed for 10 minutes in a vacuum container, followed by putting the defoamed resin mixture in a container made of Teflon (registered trademark). In the next step, the defoamed resin mixture was subjected to a preliminary curing at 85°C for one hour, followed by further curing the resin at 125°C for 2 hours so as to manufacture a raw material of the third acoustic matching layer.

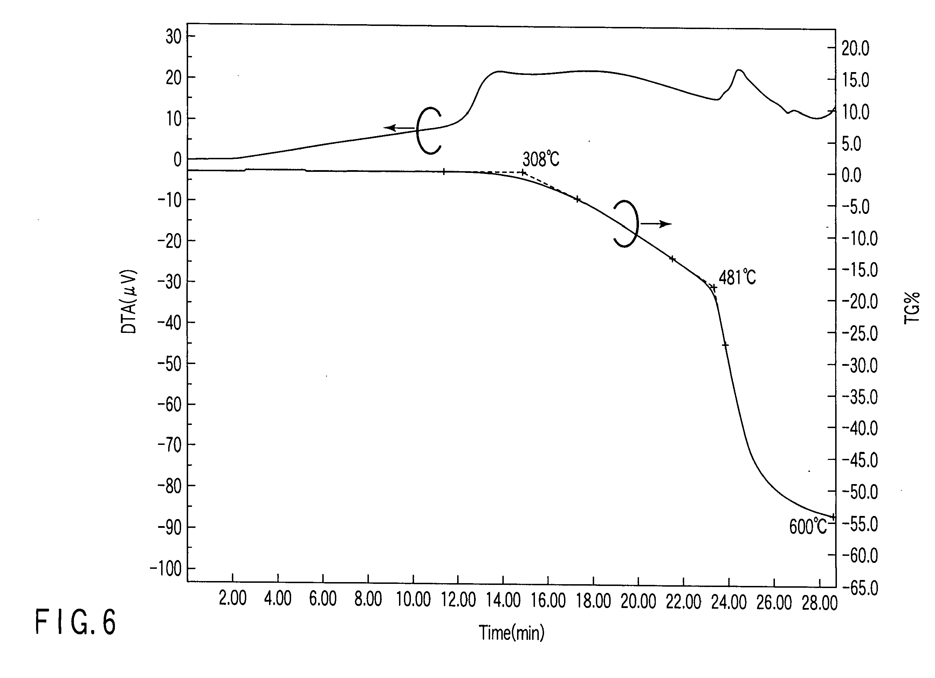

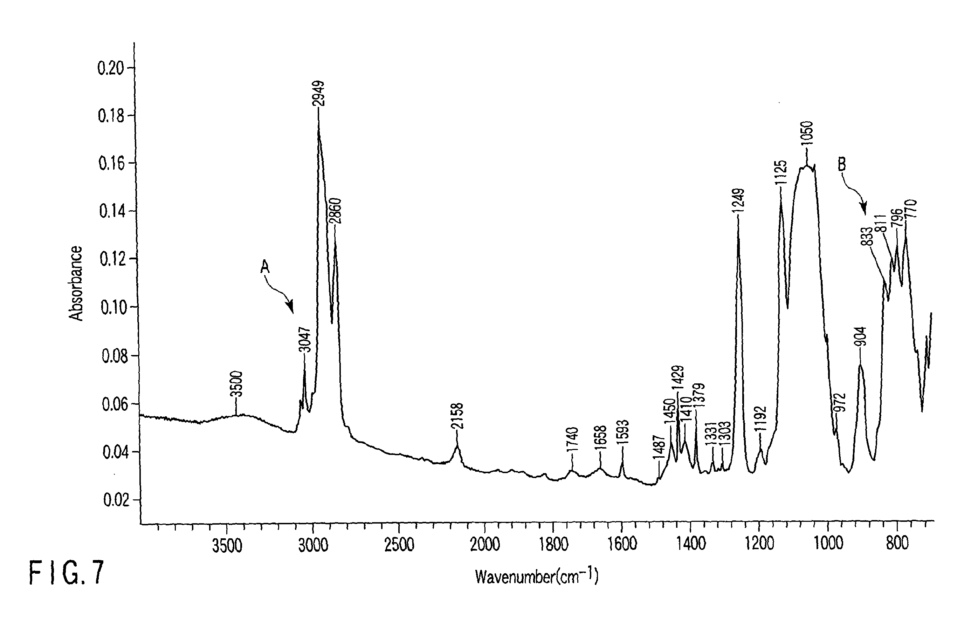

A thermogravimetric analyzer (TG) and a differential thermal analyzer (DTA) were applied to the cured material of the silicone resin-containing mixture obtained in Example 1 (raw material of the third acoustic matching layer) under the conditions given below so as to measure a TG/DTA curve. Also, a Fourier Transform Infrared Spectrophotometer (FTIR) spectrum analyzed by the FTIR was measured for the cured material of the silicone resin under the conditions below. FIG. 6 shows the TG/DTA curve, and FIG. 7 shows the FTIR spectrum.

<Conditions of TG/DTA>

Apparatus used: TG/DTA320U manufactured by SII Nano Technology Inc.;
Heating Condition: temperature elevation from room temperature to 600°C at a temperature elevation rate of 20°C/min;
Ambient gas: air stream of 200 mL/min;
Reference: sapphire 9.6 mg;
Sampling amount of a test sample: 10 mg;

<Analytical Conditions of FTIR>

Apparatus used: FTS-6000/UMA500 manufactured by Varian Technology Japan Ltd,;

Detector: MCT;
The number of times of integrating operations: 200 times;
Measuring method: Attenuated Total Reflection (ATR) method;

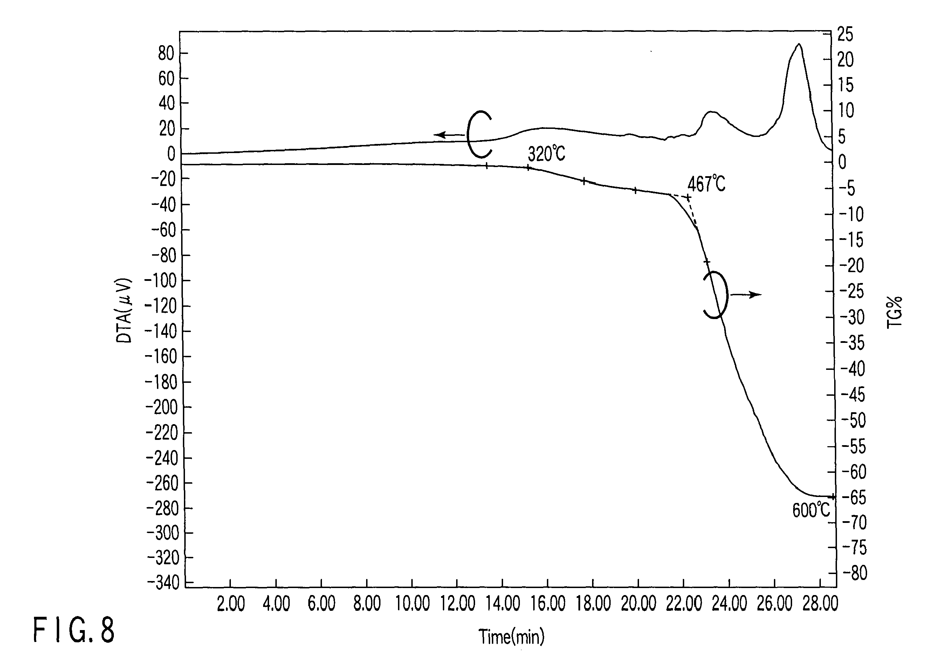

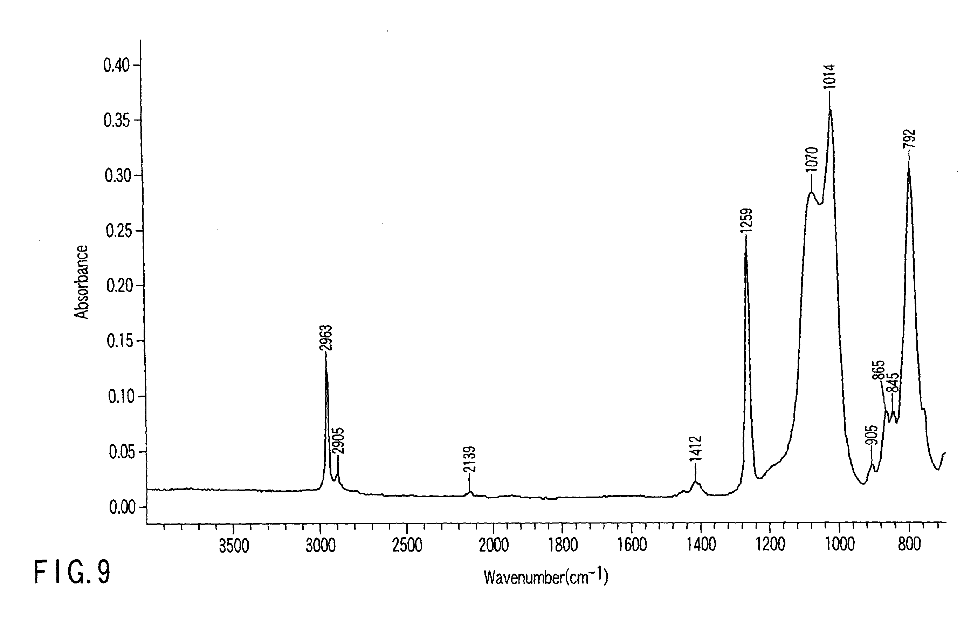

For comparison, the TG/DTA curve and the FTIR spectrum were measured under the same conditions in respect of the cured material of the general-purpose silicone resin (TSE3032 manufactured by MOMENTIVE PERFORMANCE MATERIALS Inc.). FIG. 8 shows the TG/DTA curve and FIG. 9 shows the FTIR spectrum.

As apparent from FIGS. 6 and 8, the cured material of the silicone resin-containing mixture obtained in Example 1 was found ,to exhibit the decomposition starting temperature in the second stage, which was higher than that of the cured material of the general-purpose silicone resin, and was also found to exhibit a difference of about 10% in the weight reduction, compared with the cured material of the general-purpose silicone resin.

Also, the C-H stretching vibration of benzene nucleus forming the absorbing zone derived from the benzene nucleus, which is denoted by an arrow A in FIG. 7, and the C-H outside planar angle change vibration, which is denoted by an arrow B in FIG. 7, do not appear in the cured material of the general-purpose silicone resin as apparent from FIG. 9, but appear in the cured material of the silicone resin-containing mixture obtained in Example 1 as shown in FIG. 8.

(Examples 2 to 9)

Raw materials for the third acoustic matching layers were manufactured as in Example 1 by using SCR 1012, SCR 1011 and SCR 1004, available from Shin-Etsu Chemical Co. Ltd., as two-part silicone resins (silicone resin-based mixture) as shown in Table 1. A prescribed filler was dispersed in some of these raw materials of the third acoustic matching layers. Used as the fillers were silicone rubber particles having an average particle diameter of 3 $\mu$m, epoxy resin particles having an average particle diameter of 10 $\mu$m, a powdery silicon oxide having an average particle diameter of 20 nm, a powdery zinc oxide having an average particle diameter of 30 nm, a powdery titanium oxide having an average particle diameter of 50 nm, a fibrous glass having an average diameter of 5 $\mu$m and an average length of 100 $\mu$m, and a fibrous carbon having an average diameter of 7 $\mu$m and an average length of 100 $\mu$m.

Table 1

| | Composition of third or fourth acoustic matching layer | | | | | | | |
| | Base resin | | Organic filler particle | | Powdery inorganic filler | | Fibrous inorganic filler | |
| | Kind | Amount (vol%) | Kind | Amount (vol%) | Kind | Amount (vol%) | Kind | Amount (vol%) |
| Example 1 | Silicone resin-based mixture (SCR1011) | 100 | - | - | - | - | - | - |
| Example 2 | Silicone resin-based mixture (SCR1012) | 100 | - | - | - | - | - | - |
| Example 3 | Silicone resin-based mixture (SCR1011) | 98 | - | - | Silicon oxide | 2 | - | - |
| Example 4 | Silicone resin-based mixture (SCR1011) | 95 | - | - | Silicon oxide | 2 | Glass | 3 |

(continued)

| | | Composition of third or fourth acoustic matching layer | | | | | | | |
| | | Base resin | | Organic filler particle | | Powdery inorganic filler | | Fibrous inorganic filler | |
| | | Kind | Amount (vol%) | Kind | Amount (vol%) | Kind | Amount (vol%) | Kind | Amount (vol%) |
|---|---|---|---|---|---|---|---|---|---|
| Example 5 | | Silicone resin-based mixture (SCR1011) | 90 | - | - | Silicon oxide | 5 | Glass | 5 |
| Example 6 | | Silicone resin-based mixture (SCR1011) | 80 | Silicone rubber | 10 | Silicon oxide | 5 | Glass | 5 |
| Example 7 | | Silicone resin-based mixture (SCR1011) | 90 | Epoxy resin | 5 | Zinc oxide | 5 | - | - |
| Example 8 | | Silicone resin-based mixture (SCR1004) | 90 | Silicone rubber | 8 | Titanium oxide | 2 | - | - |
| Example 9 | | Silicone resin-based mixture (SCR1004) | 70 | Silicone rubber | 28 | Silicon oxide | 1 | Carbon | 1 |

(Examples 10, 11 and Comparative Examples 1 to 6)

The raw materials of the third acoustic matching layers were manufactured as in Example 1 by using as the base resin, i.e., as the two-part silicone resin (silicone resin-based mixture), each of SCR 1011 and SCR 1012, each available from Shin-Etsu Chemical Co. Ltd., a polyurethane rubber, a silicone rubber, an epoxy resin, polyethylene, a high hardness silicone resin available from Shin-Etsu Chemical Co. Ltd under the trade name of KER 2500, and a high hardness silicone resin available from MOMENTIVE PERFORAMANCE MATERIALS Inc. under the trade name of IVSM 4500 as shown in Table 2. A prescribed filler selected from the group consisting of silicone rubber particles having an average particle diameter of 3 $\mu$m, a powdery silicon oxide having an average particle diameter of 20 nm, a powdery alumina having an average particle diameter of 100 nm and a fibrous silicon oxide having an average diameter of 5 $\mu$m and an average length of 100 $\mu$m was dispersed in some of these raw materials.

Table 2

| | Composition of third or fourth acoustic matching layer | | | | | | | |
|---|---|---|---|---|---|---|---|---|
| | Base resin | | Organic filler particle | | Powdery inorganic filler | | Fibrous inorganic filler | |
| | Kind | Amount (vol%) | Kind | Amount (vol%) | Kind | Amount (vol%) | Kind | Amount (vol%) |
| Example 10 | Silicone resin-based mixture (SCR1004) | 70 | Silicone rubber | 25 | Alumina | 3 | SSilicon oxide | 2 |
| Example 11 | Silicone resin-based mixture (DE6665) | 100 | - | - | - | - | - | - |
| Comparative Example 1 | Polyurethane rubber | 90 | - | - | Silicon oxide | 10 | - | - |
| Comparative Example 2 | Silicone rubber | 90 | - | - | Silicon oxide | 10 | - | - |
| Comparative Example 3 | Epoxy resin | 90 | - | - | Silicon oxide | 10 | - | - |
| Comparative Example 4 | Polyethylene | 100 | - | - | - | - | - | - |
| Comparative Example 5 | High hardness silicone rubber (KER2500) | 100 | - | - | - | - | - | - |
| Comparative Example 6 | High hardness silicone rubber (IVSM4500) | 100 | - | - | - | - | - | - |

The density, the acoustic velocity, the acoustic impedance (Z), the acoustic attenuation factor, the dicing machinability, the heat resistance and the adhesivity were measured as follows in respect of the raw materials of the third acoustic matching layers for each of Examples 1 to 10 and each of Comparative Examples 1 to 6.

1) Density:

The density was obtained by using a disk-like sample for evaluating the acoustic properties, which was prepared by polishing the raw material of the third acoustic matching layer, the sample having a diameter of 50 mm and a thickness of 3 mm. The density was measured by the Archimedean method by measuring the weight of the sample under an air atmosphere at 25°C and in water at 25°C.

2) Acoustic velocity and acoustic attenuation factor:

The acoustic velocity and the acoustic attenuation factor were measured by using a measuring probe of 5 MHz within water at 25°C. Used for the measurement was a disk-like sample for evaluating the acoustic properties, which was prepared by polishing the raw material of the third acoustic matching layer, the sample having a diameter of 50 mm and a thickness of 3 mm. To be more specific, ultrasonic signals were transmitted from the ultrasonic probe to a stainless steel plate disposed within water and to the sample mounted within water so as to measure the reflected echo signals.
The acoustic velocity was obtained from the difference in time between the reflected echo signals caused by the presence of the sample and from the thickness of the sample. The acoustic velocity (C) was calculated by using the formula given below based on the acoustic velocity of water under each temperature and by utilizing the difference in time of the transmitted waveform between water and the sample:

$$C = C_0 / [L - C_0 (\Delta t / d)]$$

where $C_0$ denotes the acoustic velocity of water, L denotes the distance between the ultrasonic probe and the sample (object to be measured), d denotes the thickness of the sample, $\Delta t$ denotes the difference in the zero-cross point after the initial peak in each the transmitted waveforms of water and the sample has been passed.

The acoustic attenuation factor was obtained by a prescribed method from the difference in intensity of the reflected echoes within water at 25°C, this difference being produced by the presence of the sample, and from the thickness of the sample.

3) Acoustic impedance (Z):

The acoustic impedance (Z) was obtained as the product between the density and the acoustic velocity, which were measured as described above.

4) Shore hardness:

The Shore hardness was measured in accordance with JIS K 6253 by using a Durometer type D.

5) Dicing machinability:

A PZT piezoelectric element and a sample for evaluating the dicing machinability, which was sized at 10 mm x 10 mm x 1.0 mm (thickness) and obtained by polishing the raw material of each of the third acoustic matching layers, were laminated one upon the other with an epoxy resin adhesive interposed therebetween. The piezoelectric element and the sample were laminated one upon the other on a backing prepared by adding ferrite to a chloroprene rubber. Then, the laminate structure was cut at a pitch of 100 $\mu$m toward the backing such that the backing was cut at a depth of 200 $\mu$m. The laminate structure was cut by using a diamond blade having a thickness of 50 $\mu$m, followed by turning the diamond blade by 90° so as to cut again the laminate structure at a pitch of 100 $\mu$m toward the backing such that the backing was cut at a depth of 200 $\mu$m. After the cutting stage, the remaining portion having an area of 50 $\mu$m square was observed with a microscope. In this observation, the dicing machinability was evaluated from the fall and linearity of the sample for evaluating the dicing machinability (third acoustic matching layer).

To be more specific, the dicing machinability was evaluated in four stages as given below:

A: The remaining piece of 50 $\mu$m square was quite free from problems;
B: A defect not larger than 2% was recognized in the remaining piece of 50 $\mu$m square;
C: A defect not larger than 10% was recognized in the remaining piece of 50 $\mu$m square;
D: A defect exceeding 10% was recognized in the remaining piece of 50 $\mu$m square;

6) Heat resistance:

A sample having a width of 25 mm, a length of 100 mm and a thickness of 1.6 mm was obtained by polishing the raw material of the third acoustic matching layer. The sample thus obtained was attached to a glass epoxy substrate (FR4) by using an epoxy adhesive in accordance with the method of JIS-C 6471 8.1. After the sample was cured at 60°C for 24 hours, followed by further curing the sample at 125°C for one hour, the sample was pulled at a speed of 30 cm/min by using a Tensilone type tensile tester so as to obtain a tensile shear strength. Incidentally, 10 samples were tested so as to obtain an average value of the tensile shear strength.

The heat resistance was evaluated in five stages as given below:

A: The shearing strength after the heat treatment was not lower than 2.0 N/mm$^2$;
B: The shearing strength after the heat treatment was not lower than 1.5 N/mm$^2$;
C: The shearing strength after the heat treatment was not lower than 1.0 N/mm$^2$;
D: The shearing strength after the heat treatment was not lower than 0.5 N/mm$^2$;
E: The shearing strength after the heat treatment was lower than 0.5 N/mm$^2$.

7) Adhesivity:

A sample having a width of 25 mm, a length of 100 mm and a thickness of 1.6 mm was obtained by polishing the raw material of each of the third acoustic matching layers. Then, the sample thus obtained was attached to a silicone rubber plate having a density of 1.5 g/cm$^3$ and a thickness of 5 mm, which was used as a material of the acoustic lens. An aluminum plate having a thickness of 5 mm was attached to the back surface of a silicone rubber plate, and the sample was attached to the silicone rubber plate by using "Cemedine Super X No. 8008 Clear" (registered trade mark). Then, the sample was cured at 60°C for 72 hours. Further, the cured sample was put in a heat cycle apparatus and subjected 100 times to a heat cycle between -25°C and +85°C, followed by pulling the sample by using a Tensilone type tensile tester at a speed of 30 cm/min so as to obtain the peeling strength. Incidentally, 10 test pieces were tested so as to obtain an average value of the peeling strength.
The adhesivity was measured in five stages as given below:

A: The peeling strength after the heat treatment was not lower than 1.0 N/mm$^2$;
B: The peeling strength after the heat treatment was not lower than 0.75 N/mm$^2$;
C: The peeling strength after the heat treatment was not lower than 0.5 N/mm$^2$;
D: The peeling strength after the heat treatment was not lower than 0.3 N/mm$^2$;
E: The peeling strength after the heat treatment was lower than 0.3 N/mm$^2$.

Table 3 shows the experimental data. Incidentally, the Shore hardness D of each acoustic matching layer is also shown in Table 3:

Table 3

| | Proporties of third or fourth acoustic matching layer | | | | | | | |
|---|---|---|---|---|---|---|---|---|
| | Density (g/cm$^3$) | Acoustic velocity (m/s) | Z (MRayls) | Acoustic attenuation factor (dB/cm/MHz) | Shore hardness D | Dicing machinability | Heat resistance | Adhesivity |
| Example 1 | 1.07 | 2120 | 2.27 | 2.6 | 70 | A | B | A |
| Example 2 | 1.07 | 2160 | 2.31 | 2.5 | 75 | A | B | A |
| Example 3 | 1.08 | 2120 | 2.29 | 2.8 | 72 | A | A | A |
| Example 4 | 1.14 | 2130 | 2.42 | 3.1 | 75 | A | A | A |
| Example 5 | 1.17 | 2130 | 2.49 | 3.5 | 77 | A | A | A |
| Example 6 | 1.21 | 2000 | 2.42 | 3.6 | 79 | A | A | A |
| Example 7 | 1.32 | 1890 | 2.49 | 4.7 | 79 | A | A | A |
| Example 8 | 1.05 | 2010 | 2.11 | 3.4 | 65 | A | A | A |
| Example 9 | 1.04 | 1790 | 1.86 | 3.0 | 50 | B | A | A |
| Example 10 | 1.04 | 1750 | 1.82 | 4.1 | 55 | B | A | A |
| Example 11 | 1.19 | 1760 | 2.09 | 4.6 | 60 | B | B | A |
| Comparative Example 1 | 1.09 | 1820 | 1.98 | 14.2 | <10 | D | D | D |
| Comparative Example 2 | 1.09 | 1050 | 1.14 | 4.0 | 15 | D | A | D |
| Comparative Example 3 | 1.21 | 2800 | 3.39 | 6.5 | 80 | C | B | B |
| Comparative Example 4 | 0.90 | 2400 | 2.16 | 3.5 | 30 | D | C | D |
| Comparative Example 5 | 1.07 | 1050 | 1.13 | 6.5 | 25 | B | A | A |
| Comparative Example 6 | 1.10 | 1150 | 1.27 | 5.5 | 50 | A | A | A |

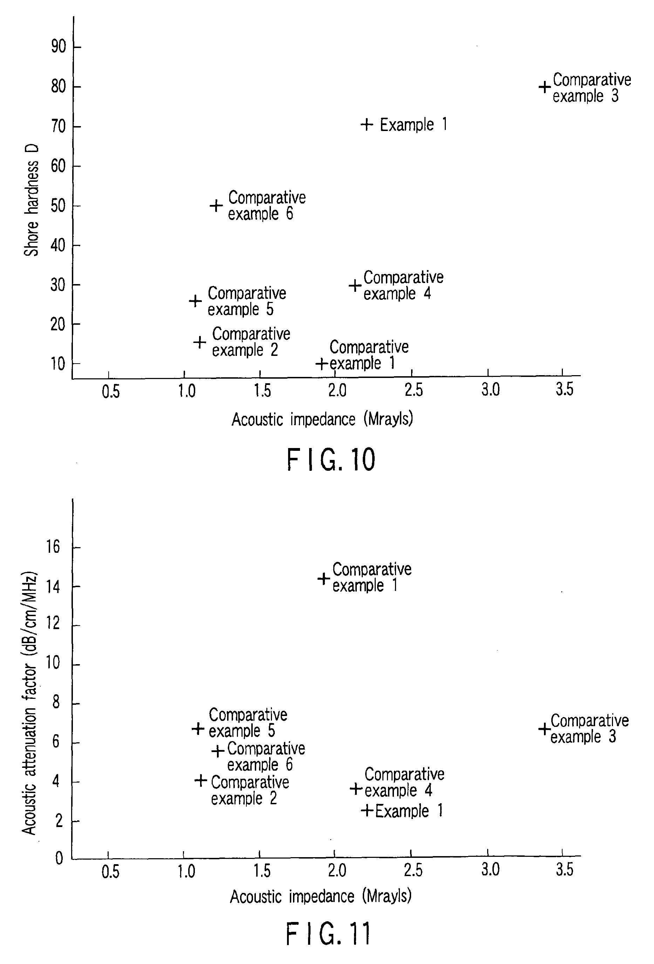

FIG. 10 is a graph showing the relationship between the acoustic impedance and the Shore hardness D in respect of the raw material of the third acoustic matching layer for Example 1 and each of Comparative Examples 1 to 5. On the other hand, FIG. 11 is a graph showing the relationship between the acoustic impedance and the acoustic attenuation factor in respect of the raw material of the third acoustic matching layer for Example 1 and each of Comparative Examples 1 to 5.

As apparent from Tables 1 to 3 and FIGS. 10 and 11, the third acoustic matching layer for each of Examples 1 to 10 was found to have a low acoustic attenuation factor and was found to be excellent in the dicing machinability and the adhesivity in spite of the fact that the acoustic impedance (Z) of the third acoustic matching layer was as low as 1.8 to 2.5 MRayls.

On the other hand, the third acoustic matching layer for each of Comparative Examples 1 to 6 was found to have a large acoustic attenuation factor or was found to be unsatisfactory in either of the dicing machinability and the adhesivity. In other words, all of the properties of the acoustic attenuation factor, the dicing machinability and the adhesivity were not satisfied in the third acoustic matching layer for each of Comparative Examples 1 to 6.

The array ultrasonic probe comprising the third acoustic matching layer for each of Examples 1 to 10 was found to be capable of effectively transmitting and receiving the energy of the ultrasonic wave so as to achieve a uniform sensitivity among the channels and to suppress the crosstalk between the adjacent channels. It follows that the array ultrasonic probe noted above was enabled to exhibit a high resolution and a good reliability over a long time.

An array ultrasonic probe including laminated acoustic matching layers of a three-layer structure was manufactured first. To be more specific, a piezoelectric element having a thickness of 400 µm was attached to a backing prepared by adding ferrite to a chloroprene rubber having an acoustic impedance (Z) of 4 MRayls. Then, a first acoustic matching layer having a thickness of 420 µm and made of a borosilicate glass was attached to the piezoelectric element, followed by attaching a second acoustic matching layer having a thickness of 200 µm and prepared by adding 20% by volume of a zinc oxide powder to an epoxy resin having an acoustic impedance (Z) of 5.0 MRayls to the first acoustic matching layer. Further, a third acoustic matching layer having a thickness of 150 µm, having an acoustic impedance (Z) of 2.29 MRayls and equal in composition to Example 3 was attached to the second acoustic matching layer. An epoxy resin based adhesive was interposed between the adjacent layers laminated on the acoustic backing. In the next step, the laminated layers were heated at 120°C for about one hour while applying pressure to the laminated layers so as to bond these laminated layers to each other. Incidentally, the piezoelectric element used included a piezoelectric body made of a PZT type piezoelectric ceramic material and first and second electrodes made of Ni and formed on both surfaces of the piezoelectric body. In the next step, a dicing process was applied from the third acoustic matching layer toward the backing so as to form trenches each having a width of 200 µm by using a diamond saw having a width of 50 µm such that the backing layer was cut at a depth of 200 µm. By this dicing process, 200 channels were formed in total, each channel consisting of two rows of the laminate structure each having a width of 200 µm. In the next step, a liquid silicone rubber was loaded in the space between the adjacent channels, followed by curing the loaded silicone rubber at 125°C for one hour. Further, an acoustic lens made of a silicone rubber and having an acoustic impedance (Z) of 1.5 MRayls was fixed to each channel with the modified silicone rubber based adhesive. Finally, the backing, the plural channels and the acoustic lens were housed in a case. Also housed in the case were a control circuit for controlling the drive timing of the piezoelectric element for each channel and a signal processing circuit including an amplifier circuit for amplifying the signal received by the piezoelectric element, thereby assembling an array ultrasonic probe of 3.5 MHz.

Also, an array ultrasonic probe including laminated acoustic matching layers of a four-layer structure was manufactured as follows. Specifically, a piezoelectric element having a thickness of 400 µm was attached to a backing prepared by adding ferrite to a chloroprene rubber having an acoustic impedance (Z) of 4 MRayls. Then, a first acoustic matching layer having a thickness of 480 µm and made of a single crystal silicon having an acoustic impedance (Z) of 19 MRayls was attached to the piezoelectric element, followed by attaching a second acoustic matching layer having a thickness of 400 µm and made of a borosilicate glass having an acoustic impedance (Z) of 10 MRayls to the first acoustic matching layer. Further, a third acoustic matching layer having a thickness of 250 µm and made of a glass-containing epoxy resin having an acoustic impedance (Z) of 4.0 MRayls was attached to the second acoustic matching layer, followed by attaching a fourth acoustic matching layer having an acoustic impedance (Z) of 2.11 MRayls and equal in composition to Example 8. An epoxy resin based adhesive was interposed between the adjacent layers laminated on the acoustic backing. In the next step, the laminated layers were heated at 120°C for about one hour while applying pressure to the laminated layers so as to bond these laminated layers to each other. Incidentally, the piezoelectric element used included a piezoelectric body made of a PZT type piezoelectric ceramic material and first and second electrodes made of Ni and formed on both surfaces of the piezoelectric body. In the next step, a dicing process was applied from the fourth acoustic matching layer toward the backing so as to form trenches each having a width of 200 µm by using a diamond saw having a width of 50 µm such that the backing layer was cut at a depth of 200 µm. By this dicing process, 200 channels were formed in total, each channel consisting of two rows of the laminate structure each having a width of 200 µm. In the next step, a liquid silicone rubber was loaded in the space between the adjacent channels, followed by curing the loaded silicone rubber at 85°C for one hour. Then, an acoustic lens made of a silicone rubber and having an acoustic impedance (Z) of 1.5 MRayls was fixed to each channel with the modified silicone rubber based adhesive. Finally, the backing, the plural channels and the acoustic lens were housed in a case. Also housed in the case were a control circuit for controlling the drive timing of the piezoelectric element for each channel and a signal processing circuit including an amplifier circuit for amplifying the signal received by the piezoelectric element, thereby assembling an array ultrasonic probe of 3.5 MHz.

Each of the array ultrasonic probes described above was connected to an image inspecting apparatus so as to evaluate the properties of the ultrasonic probe, with the result that it was found possible to obtain a good image, compared with the prior art.

Further, the array ultrasonic probe was put in a heat cycle tester for testing the heat cycle between -25°C and 85°C and the image was confirmed before and after the 100th heat cycle test. A channel defect or a decrease of the image quality was not observed.

**Claims**

1.  An array ultrasonic probe (1), comprising:

    a plurality of channels (3) arranged with spaces (4), each channel (3) including a piezoelectric element (6) and acoustic matching layers ($7_1$ to $7_3$) formed on the piezoelectric element (6) and having at least three layers;
    a backing (2) on which the piezoelectric element (6) of each channel (3) is mounted and having trenches (5) in which are formed at places corresponding to the spaces (4); and
    an acoustic lens (10) formed to cover at least the surface of the uppermost acoustic matching layer ($7_3$) of each channel (3);

    **characterized in that** the uppermost acoustic layer ($7_3$) comprises a silicone resin-containing mixture having a Shore hardness D not lower than 40 at 25°C, and exhibits an acoustic impedance of 1.8 to 2.5 MRayls.

2.  The array ultrasonic probe according to claim 1, **characterized in that** the silicone resin-containing mixture is formed of a mixture consisting of organopolysiloxane and a silicone denatured organic resin compound.

3.  The array ultrasonic probe according to claim 1, **characterized in that** the silicone resin-containing mixture is formed of a two-part mixture consisting of organopolysiloxane and a silicone series mixture containing a silicone denatured organic resin subjected to a final processing with an epoxy resin.

4.  The array ultrasonic probe according to claim 1, **characterized in that** the silicone resin-containing mixture has C-H stretching vibration of benzene nucleus and C-H outside planar angle change vibration in a spectrum obtained by a measuring of the Fourier Transform Infrared Spectrophotometer.

5.  The array ultrasonic probe according to claim 1, **characterized in that** the uppermost acoustic matching layer exhibits an acoustic attenuation factor not higher than 5 dB/cm/MHz as measured with 5 MHz at 25°C and an acoustic attenuation factor index, which is a product between the acoustic attenuation factor and the acoustic velocity and which is not larger than 900 m/s · dB/mm MHz.

6.  The array ultrasonic probe according to claim 1, **characterized in that** the uppermost acoustic matching layer exhibits a longitudinal acoustic velocity of 1750 to 2200 m/s at 25°C and has a density of 1.0 to 1.35 ($g/cm^3$).

7.  The array ultrasonic probe according to claim 1, **characterized in that** the difference in thermal expansion coefficient between the uppermost acoustic matching layer and the acoustic lens is not larger than 50 ppm/°C.

8.  The array ultrasonic probe according to claim 1, **characterized in that** the uppermost acoustic matching layer further contains at least one organic filler particles selected from the group consisting of silicone rubber particles, fluorocarbon resin particles, and urethane rubber particles in an amount not larger than 30% by volume.

9.  The array ultrasonic probe according to claim 1, **characterized in that** the uppermost acoustic matching layer further contains at least one inorganic filler selected from the group consisting of a powdery inorganic filler having a density not higher than 6 $g/cm^3$ and a fibrous inorganic filler in an amount not larger than 10% by volume.

10. The array ultrasonic probe according to claim 1, **characterized in that** the uppermost acoustic matching layer further contains at least one organic filler particles selected from the group consisting of silicone rubber particles, fluorocarbon resin particles and urethane rubber particles in an amount not larger than 30% by volume, and at least one inorganic filler selected from the group consisting of a powdery inorganic filler having a density not higher than 6 g/cm$^3$ and a fibrous inorganic filler in an amount not larger than 10% by volume.

11. The array ultrasonic probe according to claim 9, **characterized in that** the powdery inorganic filler is made of at least one material selected from the group consisting of a zinc oxide, a zirconium oxide, an alumina, a silica, a titanium oxide, a silicon carbide, an aluminum nitride, a carbon and a boron nitride.

12. The array ultrasonic probe according to claim 9, **characterized in that** the powdery inorganic filler has an average particle diameter not larger than 0.5 $\mu$m.

13. The array ultrasonic probe according to claim 9, **characterized in that** the fibrous inorganic filler is made of at least one material selected from the group consisting of a carbon, a silicon carbide, a zinc oxide, an alumina and a glass.

14. The array ultrasonic probe according to claim 9, **characterized in that** the fibrous inorganic filler has a diameter not larger than 10 $\mu$m and a length at least 5 times as much as the diameter.

15. The array ultrasonic probe according to claim 1, **characterized in that** the acoustic matching layer has a laminate structure consisting of three layers, the lower acoustic matching layer in contact with the piezoelectric element exhibits an acoustic impedance of 10 to 15 MRayls at 25°C, the intermediate acoustic matching layer exhibits an acoustic impedance of 2.7 to 8 MRayls at 25°C, and the uppermost acoustic matching layer in contact with the acoustic lens comprises a silicone resin-containing mixture having a Shore hardness D not lower than 40 and exhibits an acoustic impedance of 1.8 to 2.5 MRayls at 25°C.

16. The array ultrasonic probe according to claim 1, **characterized in that** the acoustic matching layer has a laminate structure consisting of four layers, the lower acoustic matching layer in contact with the piezoelectric element exhibits an acoustic impedance of 20 to 14 MRayls at 25°C, the second acoustic matching layer exhibits an acoustic impedance of 7 to 12 MRayls at 25°C, the third acoustic matching layer exhibits an acoustic impedance of 3 to 5 MRayls at 25°C, and the uppermost acoustic matching layer in contact with the acoustic lens comprises a silicone resin-containing mixture having a Shore hardness D not lower than 40 and exhibits an acoustic impedance of 1.8 to 2.5 MRayls at 25°C.

17. The array ultrasonic probe according to claim 1, **characterized in that** the uppermost acoustic matching layer and the acoustic lens are bonded to each other by using a rubber series adhesive exhibiting an acoustic impedance of 1.3 to 1.8 MRayls at 25°C.

18. An ultrasonic diagnostic apparatus, comprising:

an array ultrasonic probe (1) including a plurality of channels (3) arranged with spaces (4), each channel (3) having a piezoelectric element (6) and a laminated acoustic matching layer ($7_1$ to $7_3$) structure formed of at least three layers ($7_1$ to $7_3$) and arranged on the piezoelectric element (6); a backing (2) on which the piezoelectric element (6) of each channel (3) is mounted and having trenches (5) in which are formed at places corresponding to the spaces (4); and an acoustic lens (10) formed to cover at least the surface of the uppermost acoustic matching layer ($7_3$) of each channel (3), **characterized in that** the uppermost acoustic matching layer ($7_3$) comprises a silicone resin-containing mixture having a Shore hardness D not lower than 40 at 25°C, and exhibits an acoustic impedance of 1.8 to 2.5 MRayls at 25°C; and
an ultrasonic probe controller connected to the ultrasonic probe (1) via a cable (21).

FIG. 1

FIG. 2

FIG. 3

FIG. 4

FIG. 5

F I G. 6

EP 1 862 227 A2

FIG.7

F I G. 8

FIG. 9

F I G. 10

F I G. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005198261 A
- JP 10075953 A
- US 5505205 A
- JP 2004339482 A
- JP 2005272697 A

**Non-patent literature cited in the description**

- **T. INOUE et al.** *IEEE, UFFC,* 1987, vol. 34 (1), 8-15
- **TOSHIO KONDO ; HIROYUKI FUJIMOTO.** *Proceedings 2003 IEEE Ultrasonic Symposium,* 1318-1321
- **TOSHIO KONDO ; MITSUYOSHI KITATUJI ; MIKIO IZUMI.** *Proceedings, 2004 IEEE Ultrasonic Symposium,* 1659-1662